# EUROPEAN PATENT APPLICATION

(11) **EP 1 994 947 A1**
(43) Date of publication of application: **26.11.2008**
(21) Application number: 08008043.5
(22) Date of filing: 25.04.2008
(51) Int. Cl.: A61L 2/18, A61L 2/24, A61B 1/12

(54) **Endoscope washer disinfector with chemical tanks**

(30) Priority: 26.04.2007 JP 2007117530
(71) Applicant: Olympus Medical Systems Corp., Tokyo 151-0072 (JP)
(72) Inventor: Suzuki, Shintaro, Tokyo 151-0072 (JP); Suzuki, Eiri, Tokyo 151-0072 (JP); Kawachi, Shinichiro, Tokyo 151-0072 (JP)
(74) Representative: von Hellfeld, Axel

(57) **Abstract**

An endoscope washer disinfector comprises a chemical cassette, a loading unit, and a chemical tank. The chemical cassette is charged with a chemical. This chemical cassette has a delivery port to deliver the chemical therethrough. The loading unit allows the chemical cassette to be detachably loaded thereto and is formed to deliver the chemical in the chemical cassette when the chemical cassette is loaded. The loading unit comprises a blade used to forcibly open the delivery port and to sense whether or not the chemical is delivered to the chemical tank via the opened delivery port. The chemical tank stores the chemical delivered from the chemical cassette loaded to the loading unit.

## Description

### Background of the Invention

### (The field of the Invention)

The present invention relates to an endoscope washer disinfector used to wash and disinfect used endoscopes, and in particular, to an endoscope washer disinfector equipped with tanks storing chemicals for washing and disinfection.

### (Related art)

In recent years, endoscopes have been used widely in both the medical and industrial fields. Medical endoscopes have flexible tubes, which are insertable into the body of a patient and provided with imaging means. This insertion tube is also provided with one or more ducts (channels) formed therethrough. The ducts include a therapeutic-instrument insertion channel. Thus the insertion tube can be inserted into a patient's body to observe internal organs and a therapeutic instrument can be inserted into the therapeutic-instrument insertion channel to perform various treatments for the organs.

A medical endoscope needs to be inserted for examination and treatment, so that it is required to wash and disinfect the endoscope for the reuse. A technique for the washing and disinfection is to use an endoscope washer disinfector.

When this endoscope washer disinfector is used, it is enough to place the endoscope washer disinfector into the endoscope washer disinfector, in which the washer disinfector is subjected wash, disinfection, rinse and drain processes (i.e., washing and disinfection processes). In this processes, the outer surface of an endoscope and the inside of plural ducts, such as an air-supply water-supply duct and a therapeutic-instrument insertion duct are washed and disinfected.

This kind of endoscope washer disinfector uses the liquid disinfectant which is made of, for example, base agent (peracetic acid or others) and buffer agent. In general, the disinfectant is diluted at a predetermined concentration with dilution water (for example, tap water) in a disinfectant tank. This diluted disinfectant tank is used for disinfecting endoscopes.

In this endoscope washer disinfector, for preparing the disinfectant in the disinfectant tank, a first disinfectant cassette filled with the base agent and a second disinfectant cassette filled with the buffer agent are set to a loading unit. When being set, blades provided in the loading unit, cassette by cassette, break through the film seals of supply ports of the disinfectant cassettes. Thus the base agent and the buffer agent begin to flow out of the respective cassettes, and are supplied to a disinfectant tank watertightly connected to the loading unit with the aid of O-rings or similar. The amount of the flowing-out agents are sensed by plural water-level sensor placed within the disinfectant tank. Then a given amount of the dilution water is supplied to the disinfectant tank to obtain the disinfectant of a predetermined diluted concentration. In this dilution, another water-level sensor placed within the disinfectant tank senses the amount of the dilution water supplied.

That is, the amounts of the base agent, buffer agent, and dilution water supplied to the disinfectant tank are controlled using the plural water-level sensors placed within the disinfectant tank. Such sensors include an electrode sensor sensing the completion of supply of the base agent, an electrode sensor sensing the completion of supply of the buffer agent, an electrode sensor sensing the completion of supply of the dilution water, and an electrode sensor preventing the liquid from overflowing from the disinfectant tank.

In Japanese Patent Application Publication (laid-open) No. 2004-121832, an endoscope washer disinfector has a plurality of level sensors placed within a disinfectant tank. The plurality of level sensors sense stepwise the amount of the liquid contained in a disinfectant tank on the basis of the levels of the liquid. The sensed information is used to control the amounts of supply of the disinfectant and the dilution water.

Even when the amount of the disinfectant to be supplied is smaller than the capacity of the disinfectant tank, the endoscope washer disinfector disclosed by the above publication is structured such that the level sensors in the tank should sense the amounts of the liquids to be supplied into the tank. Hence reliability in determining whether or not all the disinfectant in each cassette has been supplied to the disinfectant tank is lower.

For a more precise level detection, it is required to install the level sensors in position within the disinfectant tank in a precise manner. However, this positional adjustment is not technically easier. In addition, for installing the plural level sensors within the disinfectant tank, the level sensors should be closely positioned to one another. Avoiding the positional interference among the sensors during their poisoning is troublesome work for designers.

### Summary of the Invention

The present invention has been made in consideration of the foregoing various problems, and an object of the present invention is to provide an endoscope washer disinfector which is able to reliably detect whether or not all the chemical in each chemical cassette has been supplied from the cassette to the chemical tank.

In order to achieve the above object, the present invention provides as one mode an endoscope washer disinfector comprising: a chemical cassette in which a chemical is stored and which is equipped with a delivery port through which the chemical is delivered; a loading unit to which the chemical cassette is detachably loaded and which is formed to deliver the chemical in the chemical cassette when the chemical cassette is loaded; and a chemical tank that stores the chemical delivered from the chemical cassette loaded to the loading unit via the loading unit, wherein the loading unit comprises a blade used to forcibly open the delivery port of the chemical cassette to be loaded and to sense whether or not the chemical is delivered to the chemical tank via the opened delivery port.

Thus, the present endoscope washer disinfector is able to improve accuracy in detecting whether or not all the chemical has been delivered from the chemical cassette to the chemical tank in the normal condition.

### Brief Description of the Drawings

In the accompanying drawings:
Fig. 1 is a perspective view showing an endoscope washer disinfector according to an embodiment of the present invention;
Fig. 2 is a perspective view showing the endoscope washer disinfector of which top cover is opened to show a washing/disinfecting bath for accommodating an endoscope;
Fig. 3 is a perspective view showing a loading unit arranged in the endoscope washer disinfector, disinfectant cassettes being loaded to the loading unit;
Fig. 4 is a partial sectional view showing a state in which a disinfectant cassette is set to the loading unit;
Fig. 5 is a partial sectional view showing a state in which a film seal arranged at the disinfectant delivery port of the disinfectant cassette is broken by the pair of cassette blades of the loading unit, whereby the setting is completed;
Fig. 6 is a plan view showing the loading unit connected to a disinfectant tank;
Fig. 7 is an enlarged perspective view showing the pair of cassette blades of the loading unit;
Fig. 8 is a frontal view showing the pair of cassette blades;
Fig. 9 outlines the configuration of an electrical circuit used to detect the delivery of a chemical in the endoscope washer disinfector;
Fig. 10 pictorially explains that the electrical circuit detects the delivery of the chemical by the pair of cassette blades;
Fig. 11 is a flowchart showing how to control the delivery of the chemical;
Fig. 12 is a timing chart explaining the time relation between a period of on-time of the electrode sensors provided by the pair of cassette blades and a time length of various average on-time values; and
Fig. 13 is a frontal view showing a modified pair of cassette blades.

### Detailed Description of the Preferred Embodiments

Referring to the accompanying drawings, an endoscope washer disinfector according to the present invention will now be described.

### (First embodiment)

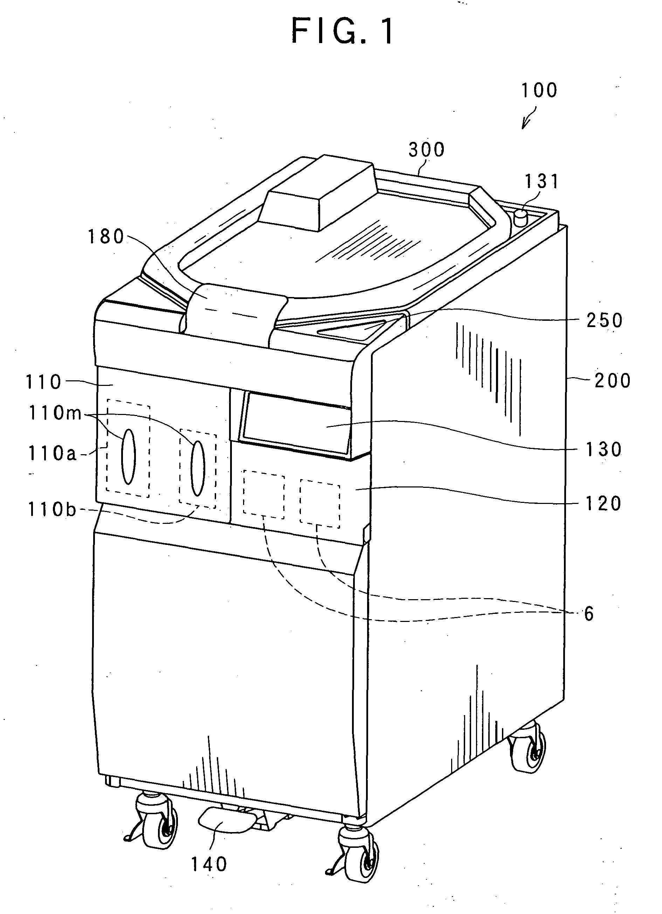

Referring to Figs. 1-12, an embodiment of the present invention will now be described.

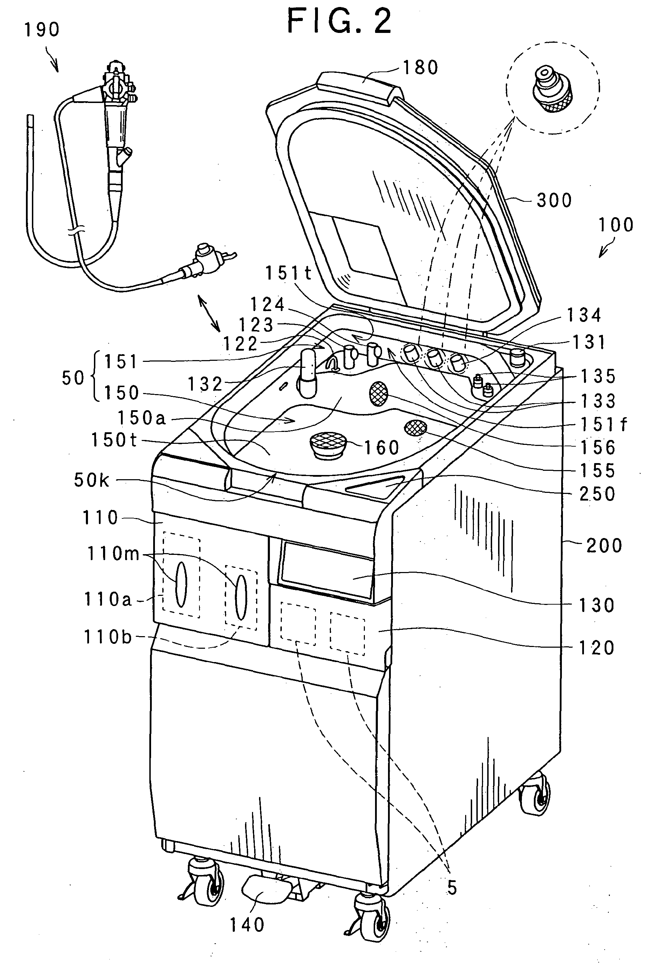

Fig. 1 shows a perspective view of an endoscope washer disinfector 100 according to the present embodiment. Fig. 2 shows a perspective view of the endoscope washer disinfector 100 with a top cover 300, in which the top cover 300 is opened to show that an endoscope 190 can be placed into the washing/disinfecting bath of the endoscope washer disinfector.

As shown in Figs. 1 and 2, the endoscope washer disinfector 100 is used to wash and disinfect the endoscope 190 which has been used. The endoscope washer disinfector 100 is equipped with an apparatus body 200 and the foregoing top cover 300 which opens and closes the top of the apparatus body 200. Hinges are used to connect the top cover 300 to the apparatus body 200.

As shown in Fig. 1, when the top cover 300 is closed on the apparatus body 200, a latch 180 fixes the top cover 300 to the apparatus body 200.

A detergent/alcohol tray 110 is set at a left-hand upper part of the front of the apparatus body 200. This tray 110 can be drawn out from the front side accessed by an operator.

In the detergent/alcohol tray 110, a detergent tank 110a and an alcohol tank 110b are contained. Liquid type of detergent used for washing the endoscope 190 is stored in the detergent tank 110a and liquid alcohol used for drying the endoscope 190 after its washing and disinfection is stored in the alcohol tank 110b. By drawing the detergent/alcohol tray 110 from the apparatus body 200, both tanks 110a and 110b can also be drawn, so that in their drawn states, an operator can refill the tanks with the detergent and alcohol.

The detergent/alcohol tray 110 is provided with two windows 110m, through which an operator can visually see the inside of the tray. The operator is thus able to confirm the residual amounts of the detergent and alcohol in the tanks 110a and 110b. In the present embodiment, the detergent is prepared as concentrated detergent, which is thus diluted at a predetermined concentration using the tap water filtered by a not-shown filter, and the mixture of the detergent and the tap water is referred to as a washing fluid.

A cassette tray 120 is provided at a right-hand upper part of the front of the apparatus body 200. This cassette tray 120 can also be drawn out of the apparatus body 200. In this cassette tray 120, two disinfectant cassettes 6 used for disinfecting the endoscope 190 are contained. These disinfectant cassettes 6 are used as chemical cassettes in which the base agent and the buffer agent of disinfectant are injected, respectively. The disinfectant is composed of peracetic acid in the present embodiment. The cassette tray 120 can be drawn from the apparatus body 120, and each disinfectant cassette 6 can be set in position in the tray 120.

At an upper part, which is higher than the cassette tray 120, of the front of the apparatus body 200, a sub-panel 130 is arranged. This panel 130 has displays showing wash/disinfection time and buttons used to command heating of the disinfectant. A pedal switch 140 is arranged at a lower part of the front of the apparatus body 200. This pedal switch 140 is operated by an operator's foot to open the top cover 300 as shown in Fig. 2.

In addition, as shown in Figs. 1 and 2, main operation panels 250 are placed at the right and left parts of the top of the apparatus body 200. These operation panels 250 are equipped with various switches including a start switch to start the washing and disinfection processes and selection switches to select a desired one from washing and disinfection modes prepared in advance.

A hose connector 131 is secured at a rear-side predetermined position on the top of the apparatus body 200. A not-shown hose connected to the tap is connected to the hose connector 131 so as to supply the apparatus body 200 with the tap water. The hose connector 131 may be equipped with a mesh filter to filter the tap water.

As shown in Fig. 2, a washing/disinfecting bath 50 is provided at an approximately central part of the top of the apparatus body 200 such that the bath 50 is opened and closed by the top cover 300. An endoscope, which has been used once, is accommodated in this bath 50 and subjected to washing and disinfection. The washing/disinfecting bath 50 includes a sink 150 and a terrace portion 151 formed to be continuous around the sink 150. The terrace portion 151 has a terrace face 151t.

The sink 150 has a bottom 150t, on which there is provided a first drain outlet 155 to discharge washing fluid, water, alcohol, disinfectant, and others delivered to the sink 150.

The sink 150 is also surrounded by a circular side face 150s and a circulation outlet 156 is provided at a given position of the side face 150s. This circulation outlet 156 is used to circulate the washing fluid, water, disinfectant and others, which have been delivered to the sink 150 via a feed/circulation nozzle 124, through the ducts (i.e., channels) of the endoscope 190. The circulation outlet 156 may have a mesh filter to filter the detergent.

The circulation outlet 156 may be provided in the bottom 150t of the sink 150. In such a configuration, the washing fluid, water, disinfectant, and others can be circulate more quickly and an operator is able to exchange the mesh filters of the circulation outlet 156 more easily.

At an approximately central part of the bottom 150t of the sink 150, there is provided a washing case 160. Buttons which are for example scope switches of the endoscope 190 and accessory parts removable from the endoscope 190 can be accommodated in the washing case 160, with the buttons and parts washed and disinfected together with the endoscope 190.

At a given position of the side face 150s, a covered water-level sensor 132 is provided to detect the water level of each of the washing fluid, water, disinfectant and others, which are delivered into the sink 150.

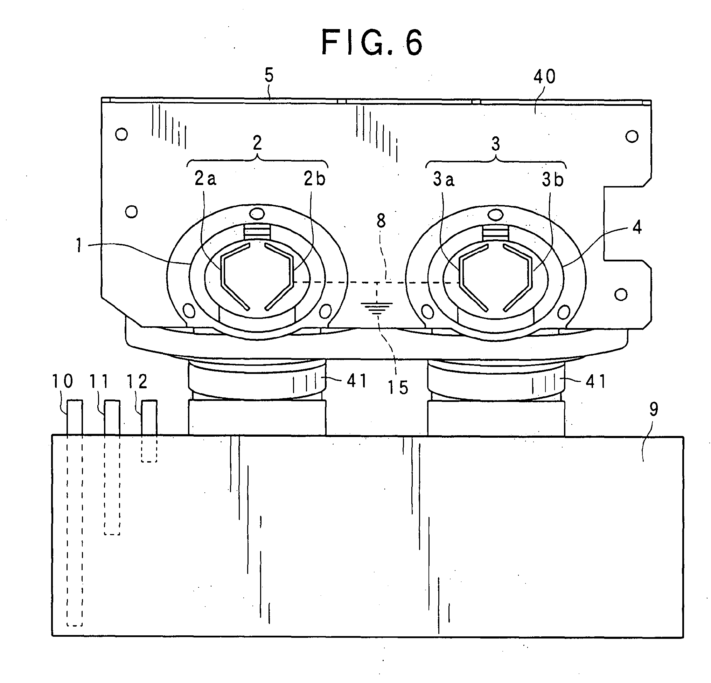

The terrace portion 151 has a step portion higher than the bottom 150t. There are provided a detergent nozzle 122, a disinfectant nozzle 123, and the feed/circulation nozzle 124. The detergent nozzle 122 is used to deliver, to the sink 150, detergent from the detergent tank 110a by means of a not-shown washing pump. The delivered detergent is diluted with the tap water, so that the washing fluid of a predetermined concentration can be obtained in the sink 150. The disinfectant nozzle 123 is used to deliver to the sink 150, using a not-shown pump, disinfectant from a disinfectant tank 9 (refer to Fig. 6) detailed later.

The feed/circulation nozzle 124 is used to feed water to the sink 150 and to supply, to the sink 150 again, the washing fluid, water, disinfectant and others which have been suctioned through the circulation outlet 156.

As a modification, the foregoing detergent nozzle 122, disinfectant nozzle 123, and feed/circulation nozzle 124 may be provided on the terrace portion 151t.

On the face 151f, which is opposed to a portion 50k of the terrace face 151t accessed by an operator, there are provided various types of ports. These ports include plural air-supply water-supply/forceps ports (in this example, two ports) for supplying the washing fluid, water, alcohol, disinfectant, and air into the duct of the endoscope 190 in addition to a forceps lifting port 134 and a water leakage detecting port 135.

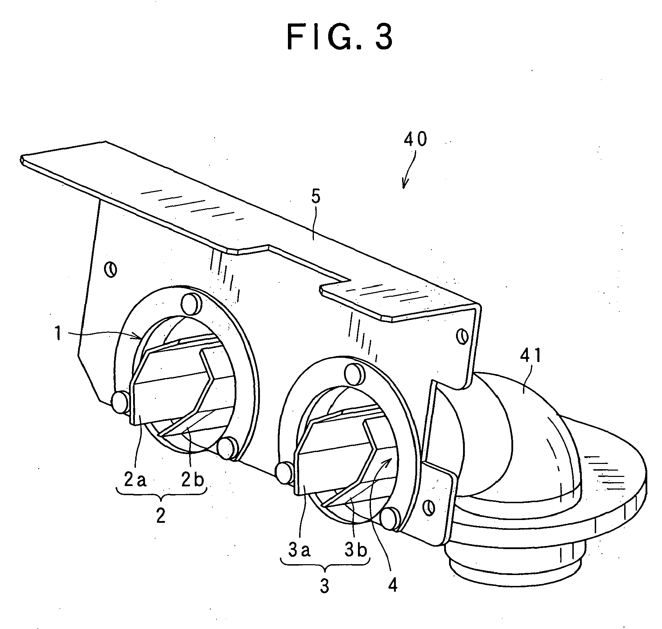

Referring to Figs. 3 - 8, the foregoing disinfectant cassette 6 will now be detailed.

Fig. 3 shows a loading unit 40 to which the two disinfectant cassettes 6 are loaded.

The loading unit 40 includes an L-shaped securing plate 5 located at an upper part of this unit 40. This plate 5 is secured to a not-shown member of the apparatus body 200, so that the loading unit 40 is arranged in position within the endoscope washer disinfector 100.

The loading unit 40 also includes two L-shaped-bent securing ducts 41, which are connected to the securing plate 5 from the rear side thereof in the state shown in Fig. 3. One end of each of the two securing ducts 41 is connected to each of the disinfectant tanks 9 (chemical tank) in a watertight manner.

The other ends of these securing ducts 41 are secured to the securing plate 5 so as to open through this plate 5. The opening of one of the securing ducts 41 serves as a base-agent cassette opening 1 to which the disinfectant cassette 6 with the base agent of the disinfectant accommodated therein is loaded. Similarly, the opening of the other of the securing ducts 41 serves as a buffer-agent cassette opening 4 to which the disinfectant cassette 6 with the buffer agent of the disinfectant accommodated therein is loaded.

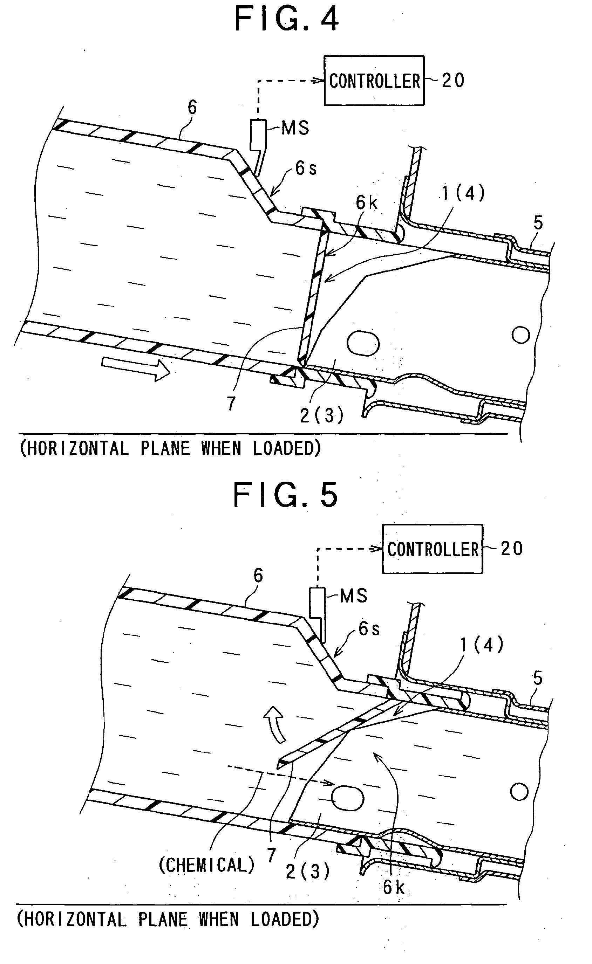

As shown in Figs. 4 and 5, the loading unit 40 is configured such that the respective disinfectant cassettes 6 are loaded to the base-agent and buffer-agent cassette openings 1 and 4 of this loading unit 40 in the state where a disinfectant delivery port 6k of each cassette 6 is directed obliquely downward. The disinfectant delivery port 6k, which serves as a chemical delivery port, is located under a loading plane 6s of each disinfectant cassette 6 to be loaded to each cassette opening. In other words, the disinfectant cassettes 6 are set obliquely to the cassette openings 1 and 4, respectively, and take an obliquely loaded attitude.

Hence, when the cassette 6 are loaded, their disinfectant delivery ports 6k are opened by a pair of cassette blades 2 and a pair of cassette blades 3, which will be detailed later. All the chemicals (base agent and buffer agent) in the disinfectant cassettes 6 are made to flow out from the cassettes due to their weight, so that no chemicals remain in the cassettes 6.

The loading unit 40 includes sensing means for sensing the fact that loading the disinfectant cassettes 6 to the cassette openings 1 and 4 is completed. In this example, the sensing means is a microswitch MS.

In the base-agent cassette opening 1, the single pair of cassette blades 2 for the base agent is provided, while in the buffer-agent cassette opening 4, the single pair of cassette blades 3 for the buffer agent is provided.

The pair of cassette blades 2 are composed of two blades disposed to not contact each other and the pair of cassette blades 3 are also composed of two blades disposed to not contact each other. As a modification, the number of cassette blades, which form a pair, is not limited to two, but may be three or more.

The pair of cassette blades 2 and the pair of cassette blades 3 have a strength which is sufficient to break a film seal 7 (refer to Fig. 4), which is provided at the disinfectant delivery port 6k of each disinfectant cassette 6 to be set to each cassette opening 1 (4). In addition, each cassette blade 2 (3) is made of an electrical conductive material, such as stainless steel, being chemical proof.

Setting the respective disinfectant cassettes 6 to the cassette tray 120 and then setting this tray 120 to the endoscope washer disinfector makes it possible to locate the cassettes 6 to face the base-agent and buffer-agent cassette openings 1 and 4, as shown in Fig. 4. After this, as shown in Fig. 5, the loading is completed by pushing the cassettes 6 to the cassette openings 1 and 4, thus allowing the pairs of cassette blades 2 and 3 to open the disinfectant delivery ports 6k, respectively.

Specifically, each of the disinfectant delivery ports 6k forcibly breaks the film seal 7, with the result that the chemicals in the disinfectant cassettes 6 flow out through the disinfectant delivery ports 6k. Each of the flowed-out chemicals is supplied from the cassette opening 1 (4) to the disinfectant tank 9 via each securing duct 41.

Each of the pairs of cassette blades 72 and 73 is located so as to come in contact with, within each cassette opening 1 (4), the chemical flowing out of each disinfectant cassette 6 loaded to this cassette opening 1 (4).

Further, as shown in Figs. 3, 6 and 7, one blade of the pair of cassette blades 2 is provided as a base-agent electrode sensor 2a, whilst the other blade is provided as a ground (GND; i.e., electrical earth) electrode sensor 2b. Similarly, one blade of the pair of cassette blades 3 is provided as a ground electrode sensor 3a, whilst the other blade is provided as a buffer-agent electrode sensor 3b.

As shown in Figs. 3, 6 - 8, of the pair of cassette blades 2, both electrode sensors 2a and 2b are symmetrical in shape to each other and located with a gap left therebetween so that an electrically non-contact state is maintained between both electrode sensors unless the chemical exits therebetween. The gap between the electrode sensors is made narrower at upper and lower positions in the base-agent cassette opening 1.

In the similar way to the above, as shown in Figs. 3, 6 - 8, the other pair of cassette blades 3 for the buffer agent is produced. That is, both electrode sensors 3a and 3b are symmetrical in shape to each other and located with a gap left therebetween so that an electrically non-contact state is maintained between both electrode sensors unless the chemical exits therebetween. The gap between the electrode sensors is made narrower at upper and lower positions in the buffer-agent cassette opening 4.

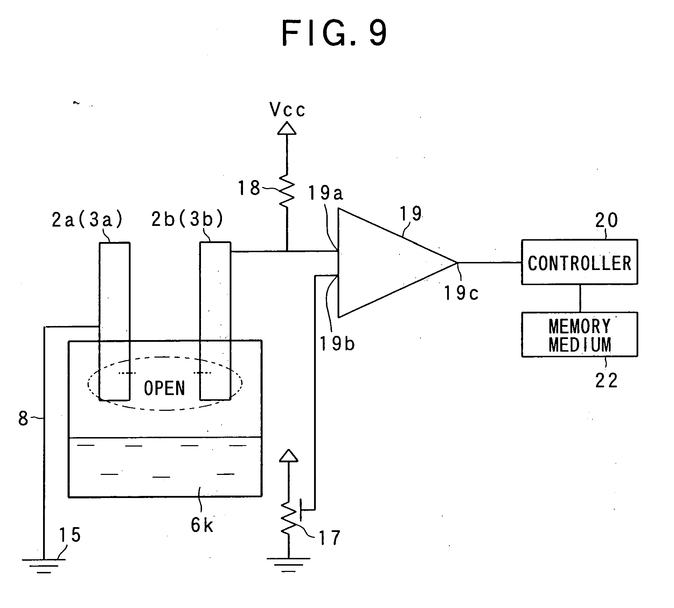

As depicted in Fig. 6, both ground electrode sensors 2b and 3a are mutually connected by a conductive wire 8 electrically connected to the ground 15. In contrast, the base-agent electrode sensor 2a and the buffer-agent electrode sensor 3b are electrically connected to a comparator 19 as shown in Fig. 9, which will be detailed later.

The base-agent electrode sensor 2a and the buffer-agent electrode sensor 3b are used to electrically sense whether or not the base agent and the buffer agent are flowing out through the cassettes' disinfectant delivery ports 6k that has been opened.

Specifically, when the base agent begins to flow out, the base-agent and ground electrode sensor 2a and 2b in the pair of cassette blades 2 are made electrically conductive therebetween via the base agent passing the port 6k. In the same way, when the buffer agent begins to flow out, the ground and buffer-agent electrode sensors 3a and 3b in the pair of the cassette blades 3 are made electrically conductive therebetween via the buffer agent passing the port 6k. Hence, by detecting changes in the voltage which occur in association with the electrical conduction, various states including the start of flow of the chemical, the intermediate step during which the chemical is still flowing, and the end of flow of the chemical (that is, the chemical has been flowed out completely) can be detected. That is, this detection utilizes information showing whether or not there is a chemical to touch the sensors 2a, 2b and 3a, 3b.

How to use the pairs of cassette blades 2 and 3 in the above detection will be detailed later.

In the loading unit 40, it is preferred that the remaining parts other then the pairs of cassette blades 2 and 3 are made of either eclectically non-conductive members or electrically conductive members whose surfaces are coated with electrically non-conductive material. This is effective for avoiding the detection-disabled state due to electrical conduction between the cassette blade pairs 2 and 3.

As shown in Fig. 6, the disinfectant tank 9 is provided with a ground electrode sensor 10, a diluting-water level detecting electrode sensor 11, and an overflow detecting electrode sensor 12.

In the disinfectant tank 9, the location of the ground electrode sensor 10 is decided in its height such that this sensor is immersed in the base agent and the buffer agent injected from the respective disinfectant cassettes 6.

The diluting-water level detecting electrode sensor 11 is located at a height where the sensor 11 detects the water level in the disinfectant tank 9 when water for diluting the base and buffer agents is injected into the tank. The detection of this water level is for controlling the amount of the diluting water to be delivered.

In cases where the diluting-water level detecting electrode sensor 11 fails to detect the water level, the overflow detecting electrode sensor 12 is activated. This electrode sensor 12 is used for preventing the disinfectant, which has been diluted to the predetermined concentration by the diluting water, from overflowing the tank 9.

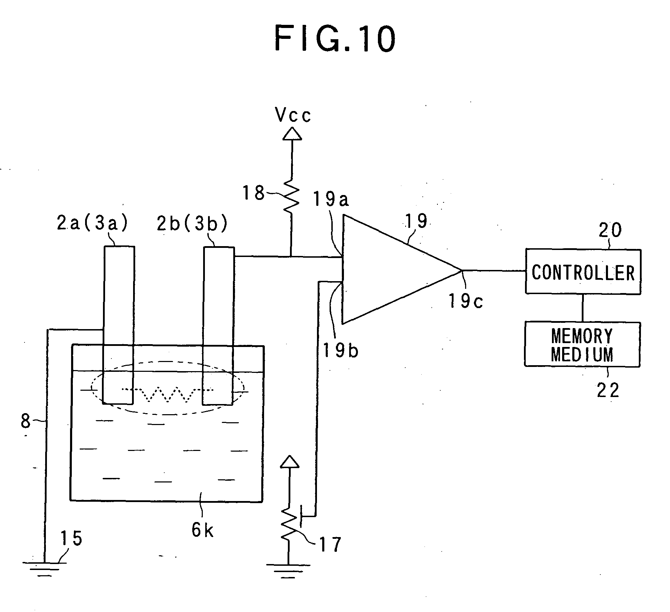

Referring to Figs. 9 and 10, how to detect the chemical in the pair of cassette blades 3 will now be described.

As shown in Fig. 9, of the pairs of cassette blades 2 and 3, the ground electrodes 2b and 3a are connected to the ground 15 by the conductive wire 8, as stated already. The base-agent electrode sensor 2a (the buffer-agent electrode sensor 3b) is electrically connected to one input terminal 19a of the comparator 19. A power-supply voltage Vcc is applied to the electrode sensor 2a (3b) and the input terminal 19a via a resistor 18.

A variable resistor 17 is electrically connected to the remaining input terminal 19b of the comparator 19 and this variable resistor 17 is connected to a not-shown power supply. Hence a reference voltage having a predetermined amount can be applied to the input terminal 19b. The comparator 19 has an output terminal 19c electrically connected to a controller 20. The controller 20 can be composed as a microcomputer with a CPU (central processing unit) and memories. The controller 20 has an electrical connection with a memory medium.

The above circuitry equipped with the comparator 19 is provided to each of the base-agent cassette opening 1 and the buffer-agent cassette opening 4.

In this circuitry, when the base-agent electrode sensor 2a and the buffer-agent electrode sensor 3b are not immersed in the chemicals as shown in Fig. 9, that is, the gap between the base agent electrode sensor 2a and the ground electrode sensor 2b and the gap between the buffer-agent electrode sensor 3b and the ground electrode sensor 3a are not subjected to the flow of the chemicals, there is no electrical conduction between the sensors 2a and 2b and between the sensors 3a and 3b. In this state, the ground electrode sensors 2b, 3a may be immersed or not immersed in the chemical.

That is, in this non-conduction state, the power-supply voltage Vcc is directly applied to the input terminal 19a of the comparator 19, so that the input terminal 19a has the voltage equal to the power-supply voltage Vcc.

In contrast, Fig. 10 shows that the base-agent electrode sensor 2a, ground electrode sensor 2b, buffer-agent electrode sensor 3b, and ground electrode sensor 3a are immersed in the respective chemicals. In this case, the base agent flows through the gap between the electrode sensors 2a and 2b and the buffer agent flows through the gap between the electrode sensors 3b and 3a. Hence the base agent allows both electrode sensors 2a and 2b to be electrically conductive and the buffer agent allows both electrode sensors 3b and 3a to be electrically conductive.

Accordingly, in this conductive state, current flows from the power-supply voltage Vcc to the ground 15, so that there occurs a drop in the voltage applied to the input terminal 19a of the comparator 19. The voltage drop depends on a ratio of the resistance of the resistor 18 and the resistance of each chemical. In other words, the voltage different from the power-supply voltage Vcc is applied to the input terminal 19a.

The comparator 19 compares the reference voltage applied to the input terminal 19b and the voltage applied to the input terminal 19a to provide high/low outputs corresponding to the presence or the absence of each chemical. When the output of the comparator 19 shows the detection of delivery of each chemical, it is recognized that the base agent and the buffer agent begins to flow or is under flow from the respective disinfectant cassettes 6. By contrast, when the output of the comparator 19 shows no detection of delivery of each chemical, it is understood that the delivery of the base agent and the buffer agent from the cassettes 6 to the disinfectant tank 9 has been completed.

The controller 20 detects outputs from the comparator 19, which outputs indicate the completion of delivery of each chemical, whereby the controller 20 determines the chemical has been accumulated up to a set water level in the disinfectant tank 9. It is preferred that the reference voltage for the comparison is changeable depending on the electrical conductivity of each chemical to be detected.

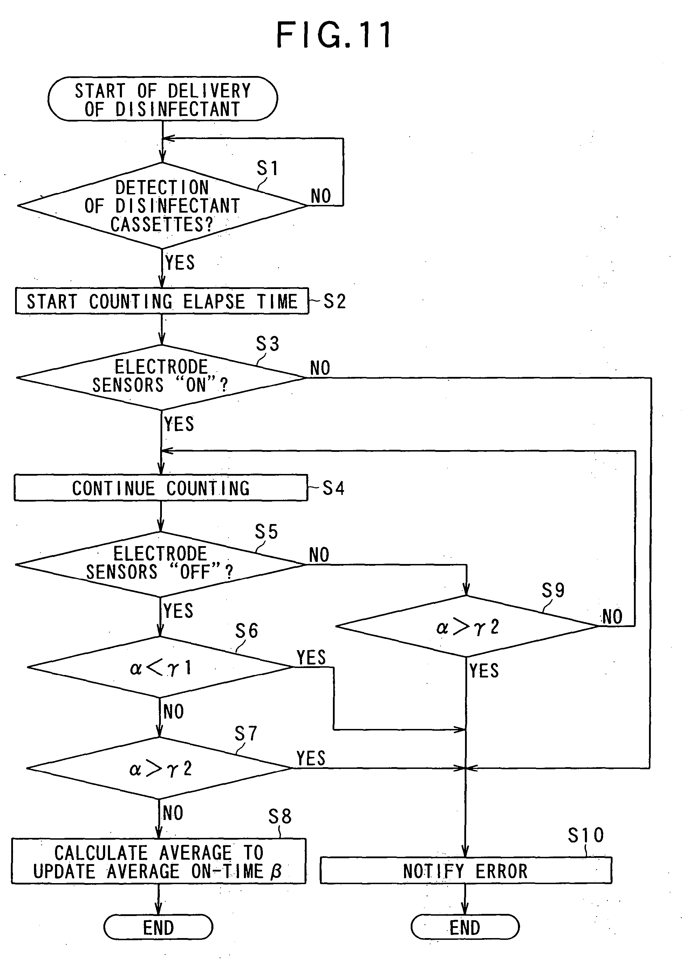

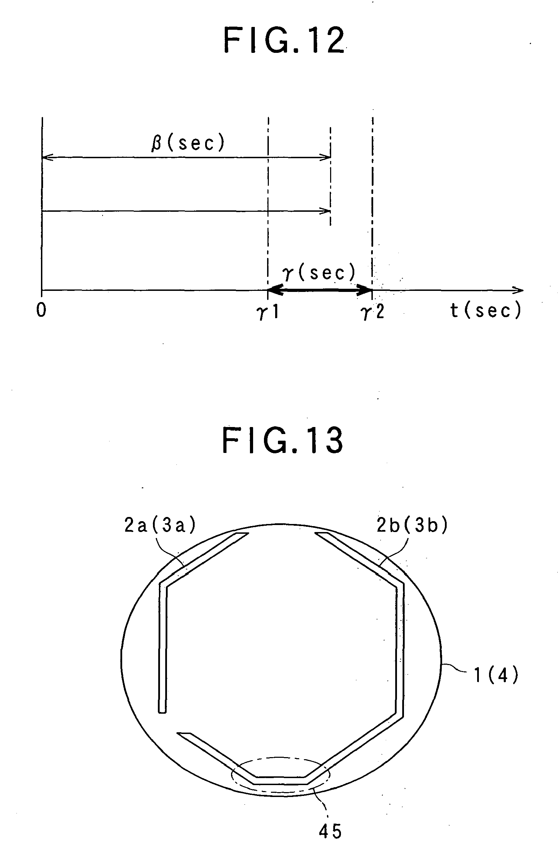

Referring to Figs. 11 and 12, how to control the delivery of the chemicals from the disinfectant cassettes 6 to the disinfectant tank 9 will now be described. This control is executed by the controller 20.

As shown in Fig. 11, at step S1, the controller 20 determines whether or not the operator has loaded the two disinfectant cassettes 6 to the base-agent and buffer-agent cassette openings 1 and 4 using the cassette tray 120.

Specifically, a signal from the microswitch MS is used to determine whether or not the film seals 7 disposed at the disinfectant delivery ports 6k of the cassettes 6 have been broken by the pairs of cassette blades 2 and 3, respectively.

When loading the disinfectant cassettes 6 is determined at step S1, the processing is shifted to step S2, where the controller 20 starts counting the elapsed time.

The processing is then shifted to step S3, where the controller 20 uses the output signals from the comparator 19 as to whether or not the base-agent electrode sensor 2a of the pair of cassette blades 2 and the buffer-agent electrode sensor 3b of the pair of cassette blades 3 are subjected to electrical conduction (i.e., ON state).

Specifically, it is determined whether or not, in the pair of cassette blades 2, the electrical conduction is established between the base-agent electrode sensor 2a and the ground electrode sensor 2b and, in the pair of cassette blades 3, the electrical conduction is established between the buffer-agent electrode sensor 3b and the ground electrode sensor 3a. The determination is made when a period of a few seconds has passed since the time count start. The reason for that is to wait for the state where the chemicals which have flowed out of the broken film seals come in contact with the pairs of cassette blades 2 and 3.

At step S3, if it is determined that the output signals of the comparator 19 indicates the ON state of the electrode sensors, the controller 20 shifts the processing to step S4 to continue counting the elapsed time, before shifting to step S5. Meanwhile if it is determined that the electrode sensors are in OFF states (non-conduction), the processing is moved to step S10, where the controller 20 recognizes the current situation as being no chemical flow from the respective disinfectant cassettes 6 and notifies the operator of an error state. After this notification, the endoscope washer disinfector 100 is brought into the standby state.

At step S5, the controller 20 determines, on the basis of the output signals from the comparator 19, whether or not the base-agent electrode sensor 2a of the pair of cassette blades 2 and the buffer-agent electrode sensor 3b of the pair of cassette blades 3 become the OFF states.

Practically, it is determined whether or not, in the pair of cassette blades 2, the electrical conduction between both base-agent and ground electrode sensors 2a and 2b has been cut off and, in the pair of cassette blades 3, the electrical conduction between both buffer-agent and ground electrode sensors 3b and 3a has been cut off.

If determination is made at step S5 such that the electrode sensors are in their OFF states, the processing is shifted to step S6, while if the determination at step S5 shows the continuation of the ON states, the processing is shifted to step S9. At step S9, it is determined whether or not a period of time α (seconds) counted from when the electrode sensors became in the ON state, which corresponds to a duration of the chemical supply, is larger than a predetermined maximum time γ2 (seconds). This time γ2 is decided as a maximum of a period of time γ (: γ1 to γ2) which is set in consideration of irregularities in an average ON-time β (seconds) of the electrode sensors. This average ON-time β is an average delivery time during which the chemicals are under delivery and can be shown as in Fig. 12. The time γ1 will be detailed later.

On the other hand, if the period of time α is equal to or smaller than the maximum time γ2, the controller 20 recognizes that the chemicals are continued being delivered from the disinfectant cassettes 6 to the disinfectant tank 9. In this case, the processing is returned to step S4 to keep counting the elapsed time.

In contrast, the period of time α is larger than the maximum time γ2, the controller 20 performs an error notification, before bringing the endoscope washer disinfector 100 into the standby state. The error is displayed, if notified, regarding various malfunctions occurring in the paths from the cassettes 6 to the tank 9. Such malfunctions include a low chemical flow rate being supplied from at least one of the two disinfectant cassettes 6, so that it will take a long time until all the chemicals flow out, and a flow-blocked state where foreign matters are jammed between the electrode sensor 2a and 2b and/or 3a and 3b.

At step S6, the controller 20 determines whether or not the period of time α is less than the minimum γ1 of the period of time γ.

If the period of time α is equal to or larger than the minimum γ1, the processing is shifted to step S7, while in the oppositely determined case, the processing is shifted to step S10 for notifying the error and making the endoscope washer disinfector into the standby state. In this case, one of presumable reasons for the error notification is inadequate breaking of the film seals 7, which results in that the chemical cannot flow out, fully, to the last drop, from at least one of the cassettes 6 so that the electrode sensors are made OFF earlier than expected.

At step S7, like the step S9, the controller 20 determines whether or not the period of time α is larger the foregoing maximum γ2.

As a result of this determination, when the period of time α is larger the maximum γ2, the controller 20 shifts the processing to step S10 for the error notification and the standby state, as stated before. One presumable reason for the error issued in this situation is that the rate of the chemical(s) flowing from the disinfectant cassettes 6 is smaller for some reason so that it takes a very long time until all the chemical(s) flows out.

At step S7, when the period of time α is equal to or less than the maximum γ2, the processing is executed at step S8, where the controller 20 determines that the chemicals have been delivered normally from the respective disinfectant cassettes 6 to the tank 9. Hence the controller 20 stores into the memory medium 22 the count showing the period of time α, and updates the current average ON-time β stored so far in the memory medium 22 on calculation with the gained count.

In the present embodiment, the film seals 7 placed at the delivery ports 6k of the respective disinfectant cassettes 6 are broken when the cassettes 6 are loaded to the loading unit 40, so that the chemicals in the cassettes 6 are delivered to the tank 9. The delivery is sensed by the electrode sensors of the pairs of cassette blades 2 and 3 disposed in the base-agent and buffer-agent cassette openings 1 and 4.

The controller 20 responds to the ON states of the chemical delivery detection so as to count the period of time α showing how long the ON states last. In addition, the controller 20 determines whether or not the period of time a is shorter than the minimum period of time γ1 or longer than the maximum period of time γ2, where the duration between the time γ1 and the time γ2 provides a time length γ taking into it consideration irregularities in the average ON-time (period of time) β. If the counted period of time α is shorter than the time γ1 or longer than the time γ2, an error state is notified and the endoscope washer disinfector itself is switched to the standby state. In addition, as long as the counted period of time α is within the time length γ, the average ON-time β stored in the memory medium 22 is updated using the counted period of time α.

In this way, the pairs of cassette blades 2 and 3, which are for breading the film seals, are used in common as the electrode sensors. Thus, unlike the conventional endoscope washer disinfector provided with the level sensors in arranged in the disinfectant tank, which has the difficulty in detecting whether or not the disinfectant chemicals are delivered from the cassettes to the tank, such a difficulty can be overcome in the present embodiment. In other words, in the present embodiment, it is possible to detect the start and end of delivery of the disinfectant chemicals more accurately.

A further advantage is to eliminate the necessity for arranging a plurality of sensors including sensors to detect the completion of delivery of the base agent and the buffer agent. Hence it is possible to reduce the number of electrode sensors which should be installed to the disinfectant tank 9. Thus it becomes easier to locate the electrode sensors secured to the disinfectant tank 9 and it is avoidable that design becomes difficult due to the fact that the plural electrode sensors should be located closely to each other to the tank 9.

Moreover, the controller 20 controls the period of delivery of the chemicals to the disinfectant tank 9. In this control, when there occurs a malfunction, an error notification is issued. When the delivery is made normally, the average delivery time β can be updated using the counted period of time α taken up by the normal chemical delivery. The control of the chemical delivery time can be made more accurately.

Accordingly, it is possible to provide the endoscope washer disinfector 100 in which easily provided are the electrode sensors sensing whether or not all the chemicals in the disinfectant cassettes 6 are delivered to the disinfectant tank 9 in the normal condition.

### (Modification)

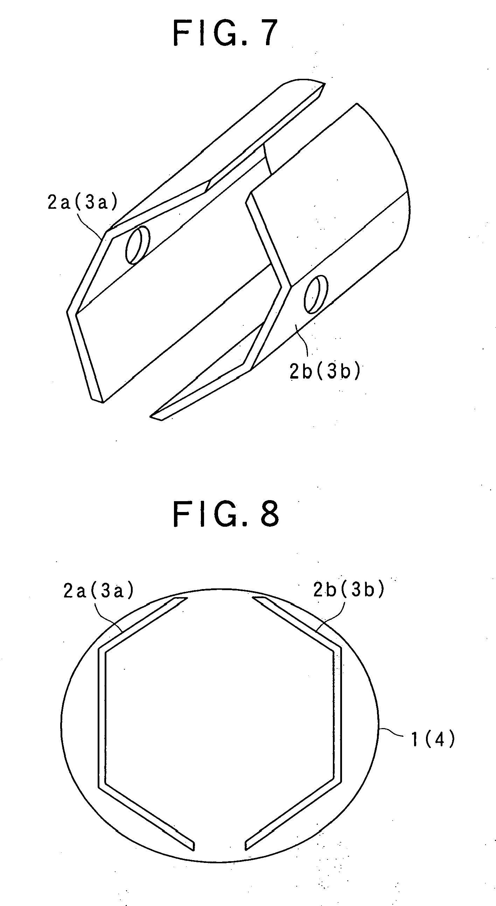

Referring to Fig. 13, a modification will now be described. Fig. 13 is a front view showing a modified form of the one pair of cassette blades shown in Fig. 8.

In the above embodiment, as shown in Figs. 6 - 8, the base-agent electrode sensor 2a and the ground electrode sensor 2b are symmetrical in shape and have a gap formed between the sensor electrodes 2a and 2b so as to make them separate form each other. The gap is made smaller in the lower and upper parts than the central part in the cassette opening 1. The other pair of cassette blades 3 has the same configuration as well. However, the present invention will not be limited to such configurations.

For example, as shown in Fig. 13, in the pair of cassette blades 2, the base-agent electrode sensor 2a and the ground electrode sensor 2b can be produced into an asymmetric shape with respect to the other to have a gap between the electrode sensors 2a and 2b. The gap has two apertures located above than the lowest part of the base-agent cassette opening 1. Hence, even if the base agent remains as a remaining fluid 45 in the lowest part of the base-agent cassette opening 1, it is possible to prevent both the electrode sensors 2a and 2b from having an erroneous electrical conduction therebetween, as easily understood from Fig. 13. This is also true of the pair of cassette blades 3.

In the foregoing embodiment, the controller 20 detects the chemicals flowing from the two disinfectant cassettes 6 using the pairs of cassette blades 2 and 3. However this is not a definitive example. Only one of the pair of cassette blades 2 and the pair of cassette blades 3 may be used to detect the base agent or the buffer agent flowing from one of the disinfectant cassettes 6.

Further, the shapes of the cassette blades 2 and 3 are also not limited to the foregoing ones. The cassette blades 2 and 3 may be produced so as to be seen as a rhombus when viewed along the longitudinal axis of each opening 1 and 4. Such rhombus-shaped blade pairs still provide the same functions as those gained by the cassette blades shown in the foregoing embodiment.

Although the descriptions above contain many specificities, these should not be construed as limiting the scope of the invention but as merely providing illustrations of some of the presently preferred embodiments of the present invention. Thus the scope of the present invention should be determined by the appended claims.

## Claims

1. An endoscope washer disinfector comprising:
a chemical cassette in which a chemical is stored and which is equipped with a delivery port through which the chemical is delivered;
a loading unit to which the chemical cassette is detachably loaded and which is formed to deliver the chemical in the chemical cassette when the chemical cassette is loaded; and
a chemical tank that stores the chemical delivered from the chemical cassette loaded to the loading unit via the loading unit,
wherein the loading unit comprises a blade used to forcibly open the delivery port of the chemical cassette to be loaded and to sense whether or not the chemical is delivered to the chemical tank via the opened delivery port.

2. The endoscope washer disinfector of claim 1, wherein
the chemical cassette comprises a sealing portion arranged at the delivery port and formed to keep the chemical within the chemical cassette when the chemical cassette is not loaded to the loading unit, and
the blade is configured to forcibly break the sealing portion when the chemical cassette is loaded to the loading unit.

3. The endoscope washer disinfector of claim 2, wherein
the blade is arranged at a predetermined position of the loading unit and the predetermined position is decided to come into contact with the chemical flowing out of the chemical cassette when the delivery port is opened.

4. The endoscope washer disinfector of claim 2, wherein
the loading unit is configured to allow the delivery port to be directed obliquely downward with the chemical cassette loaded in the loading unit, and
the delivery port is formed under a loading plane of the chemical cassette to the loading unit.

5. The endoscope washer disinfector of claim 2, wherein
the blade is made of a chemical-resistant member having a strength which is enough to open the sealing portion of the delivery port.

6. The endoscope washer disinfector of claim 5, wherein
the blade is made of a conductive material.

7. The endoscope washer disinfector of claim 6, wherein
the loading unit is made of a non-conductive material or a conductive material of which surface is coated with a non-conductive maternal, except for the blade.

8. The endoscope washer disinfector of claim 6, wherein
the blade is composed of a plurality of blades which include at least one pair of blades which are mutually non-contact to each other.

9. The endoscope washer disinfector of claim 8, wherein
the one pair of blades is composed of a blade which is in non-contact with the ground and a blade which is grounded.

10. The endoscope washer disinfector of claim 9, wherein
the chemical cassette is composed of two or more chemical cassettes and the blade corresponds in number to the two or more chemical cassettes.

11. The endoscope washer disinfector of claim 9, further comprising
a power supply that provides a voltage responding to a drop in the voltage depending on whether or not the chemical comes in touch with the non-grounded blade;
a reference power unit that provides a reference voltage; and
a comparator that compares the voltage provided by the power supply with the reference voltage to detect whether or not the chemical is delivered.

12. The endoscope washer disinfector of claim 11, wherein
the reference voltage is changeable in accordance with electrical conductivity of the chemical.

13. The endoscope washer disinfector of claim 11, further comprising a controller comprises means for counting a period of time during which the chemical is delivered from the chemical cassette to the chemical tank when the chemical cassette is loaded to the loading unit, and means for detecting start and end of the delivery of the chemical based on the counted period of time and compared results provided from the comparator.

14. The endoscope washer disinfector of claim 13, wherein
the controller comprises means for storing the counted period of time in a memory medium and updating an average delivery time of the chemical stored in the memory medium, using the counted period of time and the average delivery time currently stored in the memory medium.

15. The endoscope washer disinfector of claim 14, wherein the controller comprises means for determining whether or not the counted period of time is out of a set range in relation to the average delivery time by comparing the counted period of time and average delivery time, and means for commanding display of an error in delivering the chemical form the chemical cassette.

16. A method of delivering a chemical, which is performed by an endoscope washer disinfector provided with a loading unit to which a chemical cassette with a chemical is detachably loaded and a chemical tank to which the chemical is delivered from the chemical cassette via the loading unit when the chemical cassette is loaded to the loading unit, the method comprising:
a first step of detecting that the chemical cassette is loaded to the loading unit;
a second step of starting counting an elapsed time elapsing from the detection of loading the chemical cassette;
a third step of determining an on-state of electrode sensors in response to a touch of the electrode sensors to the chemical flowing out from the chemical cassette, the electrode sensors being provided as blades to open a delivery port of the chemical cassette, the blades being provided in the loading unit;
a fourth step of determining whether or not the electrode sensors become an off-state when a given period of time passes from the on-state of the electrode sensors; and
a fifth step of determining whether or not a chemical delivery time, which corresponds to a period of time counted during a range from the loading of the chemical cassette to the off-state of the electrode sensors, is within a given range set to an average chemical delivery time stored in a memory medium.

17. The method of claim 16, wherein
the third step includes a process to notify an error when the electrode sensors keeps the off-state thereof and bring the endoscope washer disinfector into a standby state.

18. The method of claim 17, wherein
the fourth step includes a process to i) determine whether or not the chemical delivery time is over a maximum of the given range set to the average chemical delivery time and ii) notify an error and bring the endoscope washer disinfector into a standby state when it is determined that the chemical delivery time is over the maximum, in cases where the electrode sensors are still in the on-state when the given period of time has passed.

19. The method of claim 18, wherein
the fifth step includes a process to notify an error and bring the endoscope washer disinfector into the standby state when it is determined that the chemical delivery time is out of the given range set to the average chemical delivery time.

20. The method of claim 19, wherein
the fifth step includes a process to update the average chemical delivery time stored in the memory medium on the basis of the accurately counted chemical deliver time, when it is determined that the chemical delivery time is within the given range set to the average chemical delivery time.
